(19) Europäisches Patentamt
European Patent Office
Office européen des brevets

(11) EP 2 959 857 A1

(12) EUROPEAN PATENT APPLICATION
published in accordance with Art. 153(4) EPC

(43) Date of publication:
30.12.2015 Bulletin 2015/53

(51) Int Cl.:
A61B 19/00 $^{(2006.01)}$    A61B 17/00 $^{(2006.01)}$

(21) Application number: 14753937.3

(22) Date of filing: 05.02.2014

(86) International application number:
PCT/KR2014/000979

(87) International publication number:
WO 2014/129760 (28.08.2014 Gazette 2014/35)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 21.02.2013 KR 20130018469

(71) Applicants:
• Koh Young Technology Inc.
Seoul 153-706 (KR)
• Kyungpook National University Industry-
Academic
Cooperation Foundation
Daegu 702-701 (KR)

(72) Inventors:
• HONG, Jong-Kyu
Gwangju-si
Gyeonggi-do 464-892 (KR)
• LEE, Hyun-Ki
Daegu 706-760 (KR)
• CHUNG, Jae-Heon
Gwangmyeong-si
Gyeonggi-do 423-733 (KR)
• KIM, Min-Young
Daegu 706-819 (KR)

(74) Representative: Zardi, Marco
M. Zardi & Co. SA
Via Pioda 6
6900 Lugano (CH)

(54) TRACKING SYSTEM AND TRACKING METHOD USING SAME

(57)    A tracking system and method using the same is disclosed which is capable of minimizing a restriction of surgical space by achieving a lightweight of the system as well as a reduction of a manufacturing cost through calculating a three-dimensional coordinates of each of makers using single image forming unit. In the tracking system and method using the same has an effect of reducing a manufacturing cost of the tracking system with small and lightweight, and relatively low restriction of surgical space comparing with conventional tracking system by calculating a spatial position and a direction of the markers attached on a target by using one image forming unit through a trigonometry since a pair of maker images are formed on an image forming unit for each marker by a pair of light sources positioned different to each other.

FIG. 1

EP 2 959 857 A1

**Description**

TECHNICAL FIELD

Exemplary embodiments of the present invention relate to a tracking system and tracking method using the same. More particularly, exemplary embodiments of the present invention relate to a tracking system and tracking method using the same for surgery capable of detecting a spatial position and direction information of a target by tracking coordinates of markers attached on the target, in which the target are markers attached on a patient or surgical instrument.

BACKGROUND

Recently, a robot surgery have been studied and introduced to reduce the pain of patients and to recover faster in an endoscopic surgery or an otolaryngology surgery (ENT surgery).

In such a robot surgery, in order to minimize a risk of the surgery and to operate the surgery more precisely, a navigation system is used to navigate to an exact lesion of a patient by tracking and detecting a spatial position and direction of a target such as lesion portion or surgical instrument.

The navigation system described above includes a tracking system which is capable of tracking and detecting a spatial position and direction of a target such as lesion or surgical instrument.

The tracking system described above includes a plurality of markers attached on a lesion or a surgical instrument, first and second image forming units to form images of lights emitted from the markers, and a processor calculating a three-dimensional coordinates of the markers which are coupled to the first and second image forming units and calculating a spatial position and a direction of the target by comparing pre-stored information of straight lines connecting the markers adjacent to each other and angle information formed by a pair of straight lines adjacent to each other with the three-dimensional coordinates of the markers.

Herein, in order to calculate the three-dimensional coordinates of the markers through a processor, conventionally, two detectors are required to use a triangulation method in an assumption that a coordinate of marker which is emitted from one marker and formed image in a first image forming unit and a coordinate of marker which is emitted from one marker and formed image in a second image forming unit are identical.

Thus, because conventional tracking system requires two image forming units to form images of lights which are emitted from each of the markers positioned different to each other, a manufacturing cost increases as well as a whole size also increases, and therefore a surgical space is restricted.

Therefore, the technical problem of the present invention is to provide a tracking system and method using the same capable of reducing a manufacturing cost as well as minimizing a restriction of a surgical space by achieving compact system through calculating three-dimensional coordinates for each marker using only one image forming unit.

SUMMARY

In one embodiment of the present invention, a tracking system includes at least three markers which are attached on a target to emit lights, a pair of light sources which emit lights in different position to each other, a lens portion which passes the lights emitted from the pair of the light sources and reflected by the markers, an image forming unit which forms a pair of maker images for each marker by receiving the lights which have passed by the lens portion, a processor which calculates three-dimensional coordinates of the markers by using the pair of the maker images formed on the image forming unit for each marker and calculates spatial position information and direction information of the target by comparing the three-dimensional coordinates of the markers with pre-stored geometric information between the markers adjacent to each other.

Meanwhile, the tracking system may further include a beam splitter which is arranged between the lens portion and the image forming unit to partially reflect the light, which is emitted from one light source of the pair of the light sources, toward a center of the markers, and to partially pass the light, which is emitted toward the center of the markers, re-reflected by the markers, and flowed through the lens unit, toward the image forming unit.

Also, the tracking system may further include a diffuser arranged between the light source, which emits light toward the beam splitter, and the beam splitter to diffuse the lights emitted from the light sources.

In one embodiment, the image forming unit may be a camera to form a pair of images for each marker by receiving the lights reflected by the markers and sequentially passing the lens portion and the beam splitter.

In one embodiment, the geometric information between the markers may be length information of straight lines connecting the markers adjacent to each other and angle information formed by a pair of straight lines adjacent to each other.

In one embodiment of the present invention, a tracking method includes emitting lights from a pair of light sources which are positioned at different position to each other toward at least three markers, reflecting the lights emitted from the pair of the light sources toward a lens portion by the markers, forming a pair of maker images on an image forming unit for each marker through the lights reflected from the markers and passing the lens portion, calculating three-dimensional coordinates for each marker through a processor by using the pair of maker images formed on the image forming unit for each marker, and calculating spatial position and direction of the target by comparing the three-dimensional coordinates of each marker with geometric information which is pre-stored in the processor between the markers adjacent to each other.

Herein, one light source of the light sources emits the light toward the beam splitter arranged between the lens portion and the image forming unit, and emits the light towards a center of the markers through the lens portion by partially reflecting the light by the beam splitter, and the other light source directly emits the light toward the markers.

Meanwhile, the light emitted toward the beam splitter may be emitted toward the beam splitter by a diffuser arranged between the beam splitter.

In one embodiment, the geometric information between the markers may be length information of straight lines connecting the markers adjacent to each other and angle information formed by a pair of the straight lines adjacent to each other.

In one embodiment, calculating three-dimensional coordinates of the markers may further include calculating two-dimensional central coordinates for each marker through the processor by using image formation position information of the pair of maker images formed on the image forming unit for each marker, and calculating three-dimensional coordinates of the markers by using the two-dimensional central coordinates of each marker.

Thus, according to an embodiment of the present invention, in a tracking system and tracking method using the same, one image forming unit is used to calculate spatial position information and direction information of a target by calculating three-dimensional coordinates of the markers through a trigonometry since it is possible to form a pair of maker images for each marker in different image formation positions by a pair of light sources positioned different to each other.

Therefore, there is an effect of reducing a manufacturing cost of the tracking system with small and lightweight, and relatively low restriction of surgical space comparing with conventional tracking system.

BRIEF DESCRIPTION OF THE DRAWINGS

FIG.1 is a schematic diagram of a tracking system according to an embodiment of the present invention;

FIG. 2 is an example diagram of markers attached on a target;

FIG. 3 is an example diagram explaining a method of tracking according to an embodiment of the present invention;

FIG. 4 is a block diagram explaining a method of calculating three-dimensional coordinates of markers;

FIG. 5 is an example diagram explaining an image forming state of markers by an image forming unit in case that at least three markers are arranged horizontally to a lens;

FIG. 6 is an example diagram of a change of image formation positions according to a distance between the markers and the lens portion;

FIG. 7 is an example diagram explaining a method of calculating two-dimensional central coordinates of a first marker;

FIG. 8 is an example diagram explaining a relationship between a reflection position in which light is reflected on a surface of markers and a center position of marker; and

FIG. 9 is an example diagram explaining a method of calculating a coordinate of a reflection position in which light is reflected on a surface of marker.

DETAILED DESCRIPTION

The present invention is described more fully hereinafter with reference to the accompanying drawings, in which example embodiments of the present invention are shown. The present invention may, however, be embodied in many different forms and should not be construed as limited to the example embodiments set forth herein. Rather, these example embodiments are provided so that this disclosure will be thorough and complete, and will fully convey the scope of the present invention to those skilled in the art. In the drawings, the sizes and relative sizes of layers and regions may be exaggerated for clarity.

It will be understood that, although the terms first, second, third, etc. may be used herein to describe various elements, components, regions, layers and/or sections, these elements, components, and/or sections should not be limited by these terms. These terms are only used to distinguish one element, component, region, layer or section from another region, layer or section. Thus, a first element, component, or section discussed below could be termed a second element, component, or section without departing from the teachings of the present invention.

The terminology used herein is for the purpose of describing particular example embodiments only and is not intended to be limiting of the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs.

It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, with reference to the drawings, preferred embodiments of the present invention will be described in detail.

A tracking system and method using the same according to an embodiment of the present invention attaches at least three markers at a target such as a lesion or a surgical instrument and calculates three-dimensional coordinates of the markers, compares geometric information of markers adjacent to each other which are pre-stored in a processor with the three-dimensional coordinates of the markers through the processor, and therefore, is capable of calculating spatial position information and direction information of a target such as a lesion or surgical instrument. The detailed description is explained referencing figures.

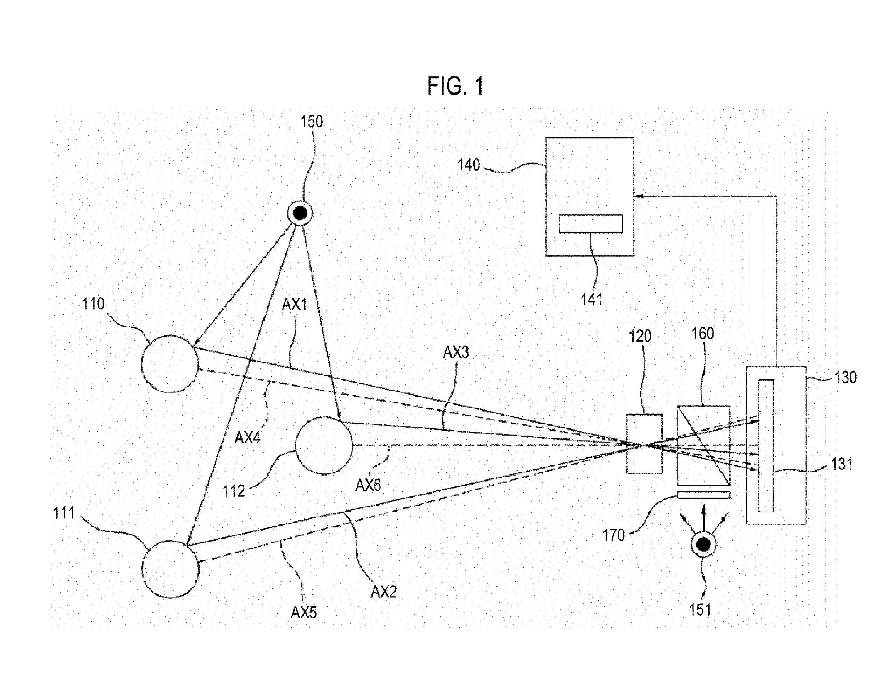

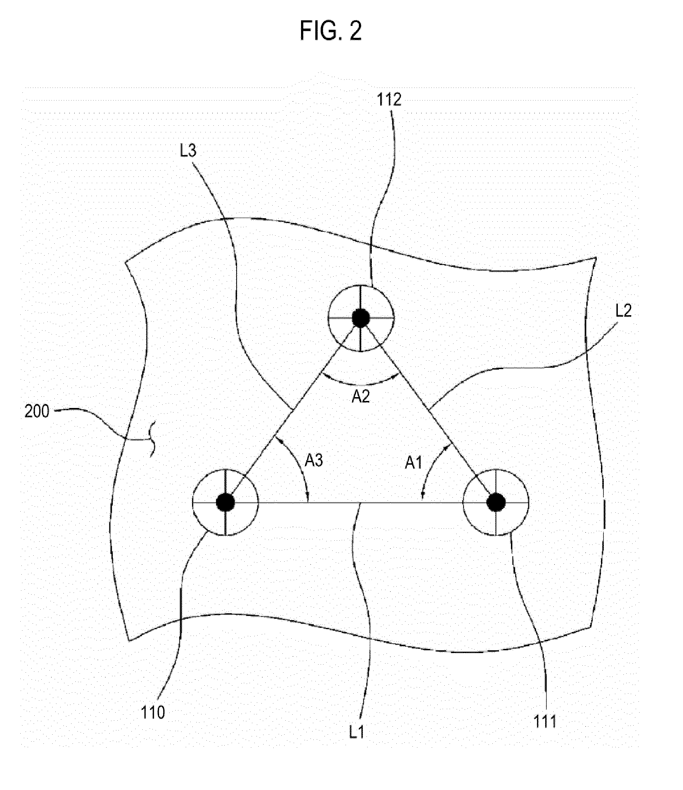

FIG.1 is a schematic diagram of a tracking system according to an embodiment of the present invention, and FIG. 2 is an example diagram of markers attached on a target.

Referring to FIGS. 1 and 2, a tracking system according to an embodiment of the present invention includes at least three markers 110, 111, and 112, at least two light sources, a first light source 150 and a second light source 151, a lens portion 120, a beam splitter 160, an image forming unit 140, and a processor 140.

The at least three markers 110, 111, and 112 are attached on a target 200 such as a lesion of a patient or a surgical instrument. Herein, the at least three markers 110, 111, and 112 are separated to adjacent markers 110, 111, and 112 to each other at a predetermined interval and the markers are attached on the target 200 such as a lesion or a surgical instrument such that a pair of straight lines L1, L2, and L3 in which each of the markers are virtually connected to adjacent markers forming specific angles A1, A2, and A3.

Herein, geometric information between the markers 110, 111, and 112 which are adjacent to each other, in other words, length information of straight lines L1, L2, and L3 which connect the markers 110, 111, 112 which are adjacent to each other and angle information A1, A2, and A3 which are formed by a pair of the straight lines connecting the adjacent markers, are stored in a memory 141 of the processor 140.

For example, the markers 110, 111, and 112 may be attached on the target 200 such as a lesion and a surgical instrument in a triangle shape, length information of straight lines L1, L2, and L3 forming sides of the triangle in which the markers are used as vertices and angle information A1, A2, and A3 which are formed by a pair of adjacent and virtual straight lines which connect the markers 110, 111, and 112 may be pre-stored in the memory 141 included in a processor 140.

Meanwhile, the markers 110, 111, and 112 may be passive markers which reflect the lights emitted from the first and second light sources 150 and 151.

The first and second light sources 150 and 151 emit the lights toward the markers 110, 111, and 112 at different position to each other. For example, the first light source 150 may directly emit the light toward the markers 110, 111, and 112, and the second light source 151 may emit the light toward the beam splitter 160, which is arranged between the lens portion 120 and the image forming unit 130. The light emitted from second light source 151 is partially reflected by the beam splitter 160, and is emitted toward a center of the markers 110, 111, and 112 after passing the lens portion 120.

In other words, a portion of the light emitted from the second light source 151 is reflected by the beam splitter 160 and emitted toward the center of the markers 110, 111, and 112 through the lens portion 120, and the rest of the light passes the beam splitter 160.

Alternatively, the second light source 151 may directly emit the light toward the makers 110, 111, and 112, and the first light source 150 may emit the light toward the beam splitter 160 such that the light is emitted toward the center of the markers 110, 111, and 112 through the beam splitter 160.

For example, it may be preferable to use a spot illumination as the first and second light sources 150 and 151 such that the lights are reflected at one point within the whole surface of the markers 110, 111, and 112.

The lens portion 120 passes the lights which is directly reflected by the markers 110, 111, and 112, which is emitted from one of the selected light source among the first and second light sources 150 and 151 and the re-reflected light which is emitted from the other light source, reflected by the beam splitter 160, and emitted toward the center of the markers 110, 111, and 112 through the lens potion 120.

The beam splitter 160 is arranged on a rear portion of the lens portion 120. The beam splitter 160 partially passes the light emitted from one light source which is selected among the first and second light sources 150 and 151, and reflects and emits the remaining light toward the center of the markers 110, 111, and 112 after passing the lens portion 120.

The image forming unit 130 is arranged on a rear portion of the beam splitter 160 and forms a pair of marker images for each of the markers 110, 111, and 112 by receiving the light that have sequentially passed the lens portion 120 and the beam splitter 160.

In other words, the image forming unit 130 is arranged on a rear portion of the beam splitter 160 and forms a pair of maker images for each of the markers 110, 111, and 112 by receiving the lights which is directly emitted from one light source which is selected among the first and second light sources 150 and 151, reflected by the markers 110, 111, and 112, and have sequentially passed the lens portion 120 and the beam splitter 160, and the light which is emitted from the other light source toward the beam splitter 160, reflected by the beam splitter 160 and have passed the lens portion 120, emitted and re-reflected toward the center of the markers 110, 111, and 112, and have sequentially passed the lens portion 120 and the beam splitter 160.

Herein, a portion of the light which is emitted from the first and second light sources 150 and 151, re-reflected by the markers 110, 111, and 112 and have passed the lens portion 120 and flowed in the beam splitter 160, is reflected by the beam splitter 160 and the rest of the light is flowed into the image forming unit 130 after passing the beam splitter 160 and forms a pair of maker images for each of the markers 110, 111, and 112.

For example, the image forming unit 130 may be a camera integrating an image sensor 131 which forms a pair of maker images for each of the markers 110, 111, and 112 by receiving the lights which are emitted from the first and second light sources 150 and 151, reflected by the markers 110, 111, and 112, and sequentially have passed the lens portion 120 and the beam splitter 160.

Meanwhile, a diffuser 170 may be arranged between the light source which emits light toward the beam splitter 160 among the first and second light sources 150 and 151 and the beam splitter 160 to diffuse the light toward the beam splitter 160.

The processor 140 calculates three-dimensional coordinates of the markers 110, 111, and 112 by using the pair of the maker images for each of the markers 110, 111, and 112 which are formed on the image forming unit 130, in which the lights are emitted from the first and second light sources 150 and 151, reflected by the markers 110, 111, and 112 and are formed on the image forming unit 130, and calculates spatial position information and direction information of the markers 110, 111, and 112 which are attached on the target 200 such as a lesion or a surgical instrument by comparing the three-dimensional coordinates of the markers 110, 111, and 112 with geometric information between the markers 110, 111, and 112 adjacent to each other.

Herein, the memory 141 is integrated in the processor 140. Meanwhile, the memory 141 integrated in the processor 140 may pre-store geometric information of the markers 110, 111, and 112 adjacent to each other, in other words, length information of straight lines L1, L2, and L3 connecting the markers 110, 111, and 112 adjacent to each other, and angle information A1, A2, and A3 formed by a pair of the straight lines connecting the markers 110, 111, and 112 adjacent to each other.

In addition, the memory 141 integrated in the processor 140 may pre-store spatial position information and direction information of the first and second light sources 150 and 151.

Referring to FIGS. 1-8, a tracking process of spatial position information and direction information of a target using a tracking system according to an embodiment of the present invention is described below.

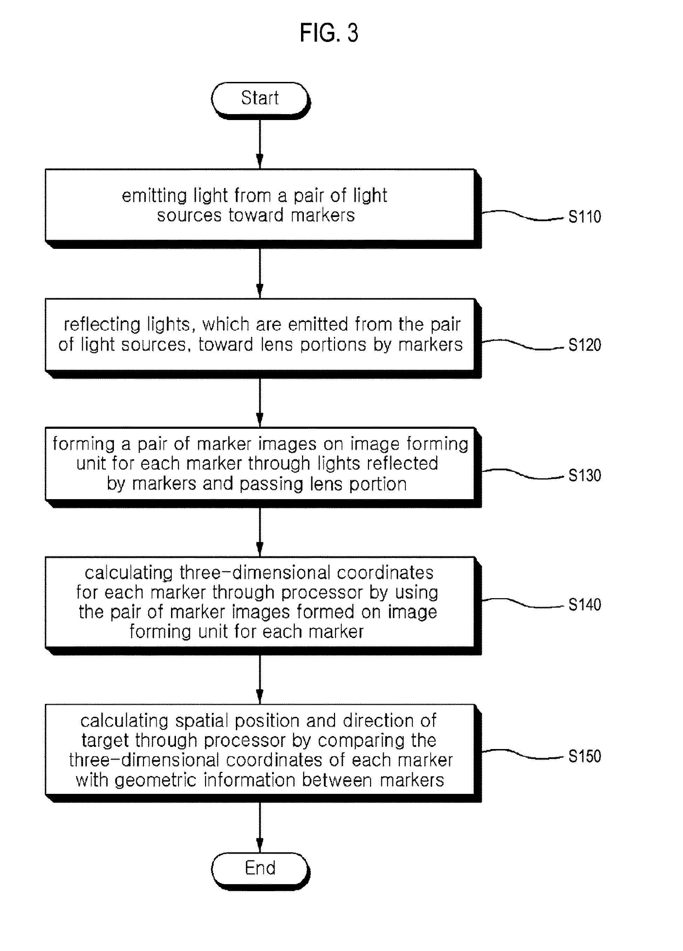

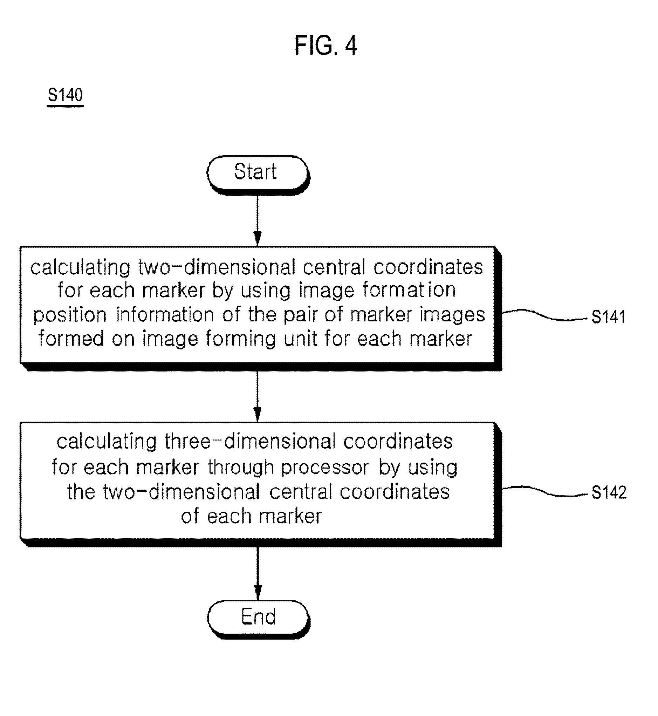

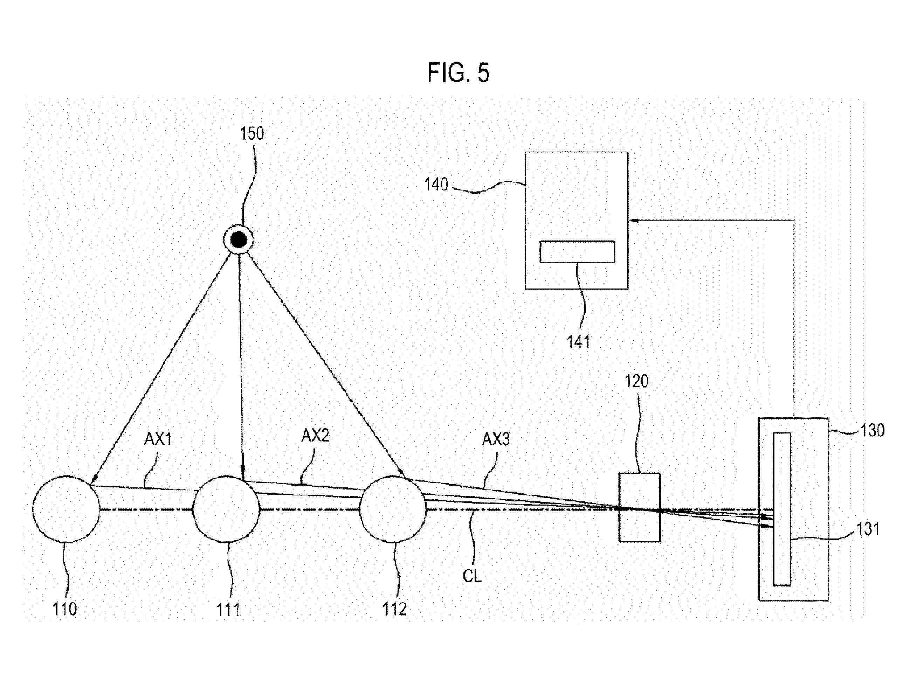

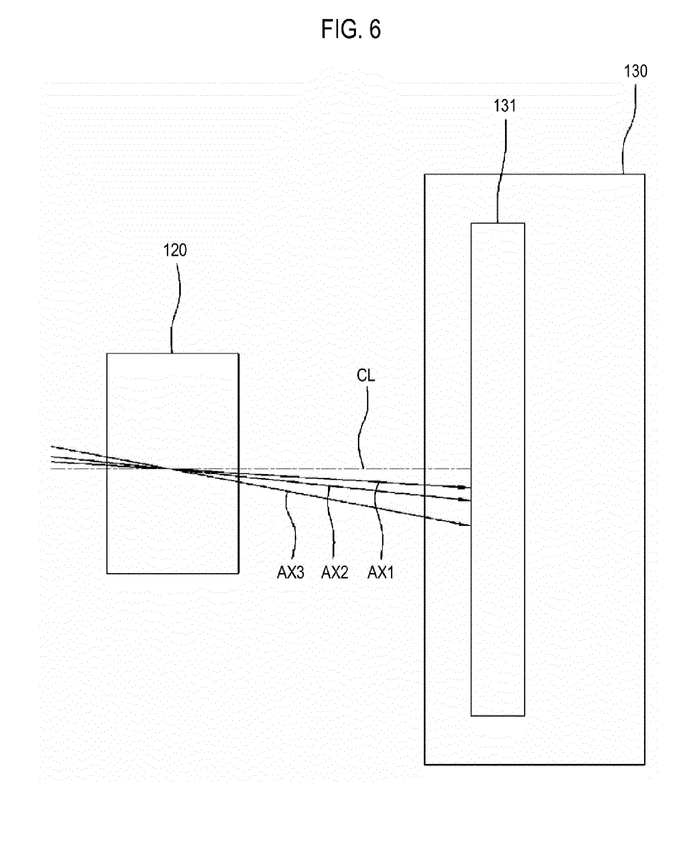

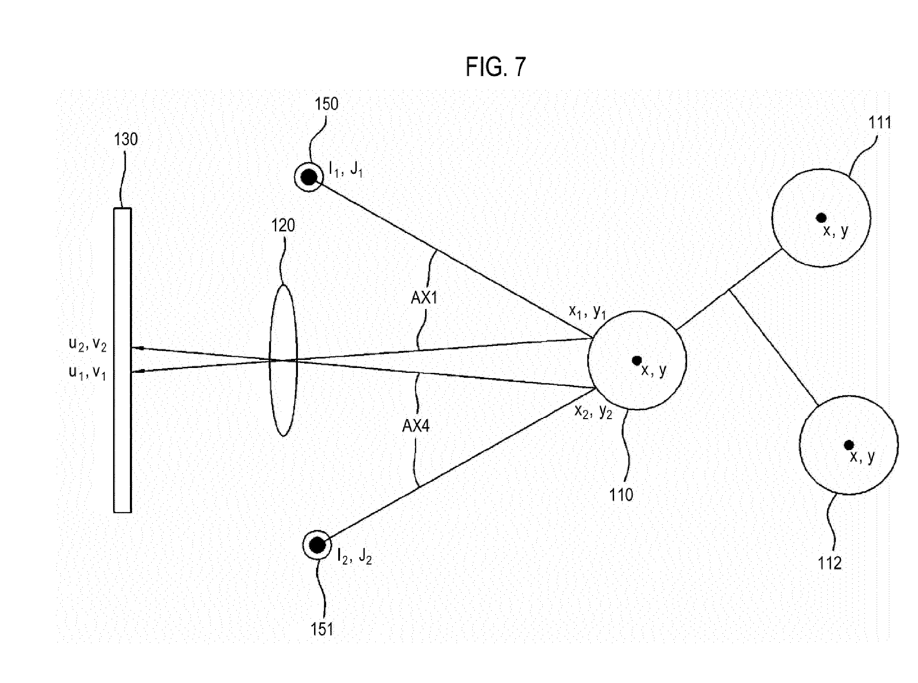

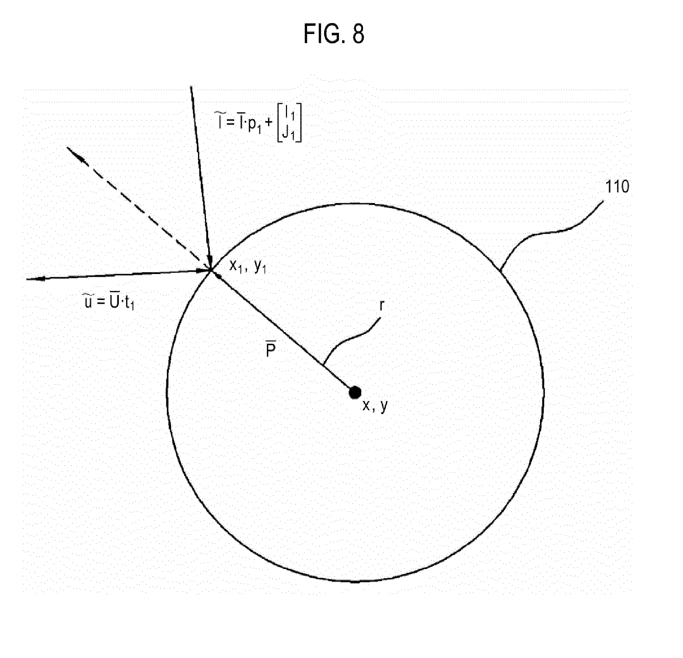

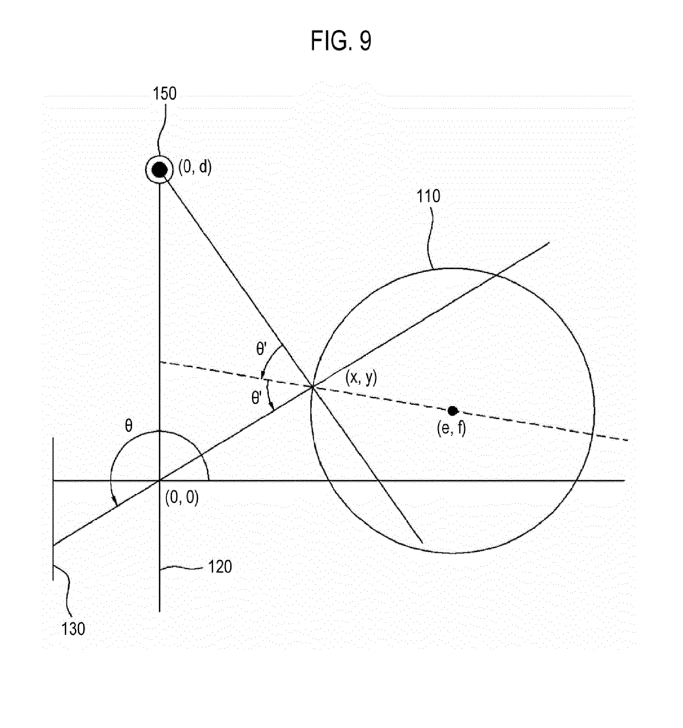

FIG. 3 is an example diagram explaining a method of tracking according to an embodiment of the present invention; FIG. 4 is a block diagram explaining a method of calculating three-dimensional coordinates of markers; FIG. 5 is an example diagram explaining an image forming state of markers by an image forming unit in case that at least three markers are arranged horizontally to a lens; FIG. 6 is an example diagram of a change of image formation positions according to a distance between markers and a lens portion; FIG. 7 is an example diagram explaining a method of calculating two-dimensional central coordinates of a first marker; FIG. 8 is an example diagram explaining a relationship between a reflection position in which light is reflected on a surface of marker and a center position of marker; and FIG. 9 is an example diagram explaining a method of calculating a coordinate of a reflection position in which light is reflected on a surface of marker.

For the convenience of explanation, it will be described as an example in the case that a first light source is arranged to directly emit a light toward to markers, and a second light source is arranged to emit a light toward the markers through a beam splitter.

Referring to FIGS. 1-9, in order to track spatial position information and direction information of a target using a tracking system according to an embodiment of the present invention, at first, the first and second light sources 150 and 151 positioned different to each other are operated to emit lights toward a first to third markers 110, 111, and 112 (S110).

In more detail, a spot illumination emitted from the first light source 150 are directly irradiated toward the first to third markers 110, 111, and 112, and a spot illumination emitted from the second light source 151 is irradiated toward a beam splitter 160 such that a portion of the light passes the beam splitter 160 and the remaining light is reflected by the beam splitter 160, passes a lens portion 120, and emits toward a center of the first to third markers 110, 111, and 112.

The spot illuminations emitted from the first and second light sources 150 and 151, which are emitted toward the markers 110, 111, and 112, are reflected toward a lens portion 120 by the first to third markers 110, 111, and 112 (S120).

In more detail, the light emitted from the first light source 150 is directly reflected at one position of a surface of the first to third markers 110, 111, and 112, reflected toward the lens portion 120 through a first to third optical paths AX1, AX2, and AX3, the light emitted from the second light source 151 is irradiated toward the beam splitter 160, a portion of the light passes the beam splitter 160 and the remaining is reflected by the beam splitter 160, passes the lens portion 120 through a fourth to sixth optical paths AX4, AX5, and AX6, and emits toward the first to third markers 110, 111, and 112.

The lights which pass the lens portion 120 through the first to sixth optical paths AX1, AX2, AX3, AX4, AX5, and AX6 form a pair of maker images on an image forming unit 130 for each of the markers 110, 111, and 112 (S130).

In more detail, the light which is emitted from the first light source 150, reflected by the first marker 110 through the first optical path AX1 and have passed the lens portion 120, forms a first image of the first marker 110 on the image forming unit 130, and the light which is emitted from the second light source 151, reflected by the first marker 110 through the fourth optical path AX4 and have passed the lens portion 120, forms a second image of the first marker 110 on the image forming unit 130. Also, the light which is emitted from the first light source 151, reflected by the second marker 111 through the second optical path AX2 and have passed the lens portion 120, forms a first image of the second marker 111 on the image forming unit 130, and the light which is emitted from the second light source 151, reflected by the second marker 111 through the fifth optical path AX5 and have passed the lens portion 120, forms a second image of the second marker 111 on the image forming unit 130. Also, the light which is emitted from the first light source 150, reflected by the third marker 112 through the third optical path AX3 and have passed the lens portion 120, forms a first image of the third marker 112 on the image forming unit 130, and the light which is emitted from the second light source 151, reflected by the third marker 112 through the sixth optical path AX6 and have passed the lens portion 120, forms a second image of the third marker 112 on the image forming unit 130.

As described above, after forming the first and second images of the first to third markers 110, 111, and 112 on the image forming unit 130, three-dimensional coordinates of the first to third markers 110, 111, and 112 are calculated by using the first and second images of the first to third markers 110, 111, and 112 formed on the image forming unit 130 through a processor 140 (S140).

Detailed explanation of calculating the three-dimensional coordinates of the first to third markers 110, 111, and 112 is described below.

In order to calculate the three-dimensional coordinates of the first to third markers 110, 111, and 112, first, two-dimensional central coordinates are calculated through the processor 140 by using image formation position information of the first and second images of the first to third markers 110, 111, and 112 formed on the image forming unit 130 (S141).

Calculating the two-dimensional central coordinates of each of the markers 110, 111, and 112 is explained in detail. For the convenience of explanation, in FIGS. 5 and 6, it will be described as an example in the case that the first to third markers 110, 111, and 112 are arranged horizontally to the lens portion. And the beam splitter is omitted in the figure as it is explaining the case that the first to third markers 110, 111, and 112 are arranged horizontally to the lens portion.

Also, as shown in FIG. 1, image formation positions of the second images of the first to third markers 110, 111, and 112 is omitted since they are identical to a center line CL of the lens portion 120 in FIGS. 5 and 6 , in which the second images are formed by the lights emitted from the second light source 151 toward the center of each of the markers 110, 111, and 112, reflected by the markers 110, 111, and 112, and flowed into the image forming unit 130 through the fourth to sixth optical paths AX4, AX5, and AX6.

As shown in FIGS. 5 and 6, the light emitted from the first light source 150 is reflected in different position of surfaces of each of the markers 110, 111, and 112 through the first to third optical paths AX1, AX2, and AX3, and forms images on the image forming unit 130. Therefore, the first images of the first to third markers 110, 111, and 112 are formed in different positions to each other, in which the first images of the first to third markers 110, 111, and 112 are formed by the light flowed in to the lens portion 120 which is emitted from the first light source 150, reflected in different position of surfaces of each of the markers 110, 111, and 112 through the first to third optical paths AX1, AX2, and AX3.

Therefore, the two-dimensional central coordinates of each of the markers 110, 111, and 112 are calculated through the processor 140 by using the image formation position information of the first images of the first to third markers 110, 111, and 112, the image formation position information of the second maker images, the position information of the first and second light sources 150 and 151 which are pre-stored in the processor 140, and radius information of the first to third markers 110, 111, and 112.

The process of calculating the two-dimensional central coordinates of the markers by the processor is described below.

As shown in FIG. 7, the two-dimensional central coordinates of the markers 110, 111, and 112 is defined as x, y, a coordinate of a first light source 150 is defined as $I_1$, $J_1$, a coordinate of a second light source 151 is defined as $I_2$, $J_2$, a coordinate of a first image which is emitted from the first light source 150, reflected by a fist marker 110 and forming an image on an image forming unit 130, is defined as $U_1$, $V_1$, a coordinate of a second image which is emitted from the second light source 151, reflected by a fist marker 110 and forming an image on an image forming unit 130, is defined as $U_2$, $V_2$, a coordinate of a reflection position, in which the light emitted from the first light source 150 and reflected by the first marker 110, is defined as $x_1$, $y_1$, a coordinate of a reflection position, in which the light emitted from the second light source 151 and reflected by the first marker 110, is defined as $x_2$, $y_2$, a vector $\overline{U}$ from $U_1$, $V_1$ to $x_1$, $y_1$, a vector $\overline{V}$ from $U_2$, $V_2$ to $x_2$, $y_2$, a vector $\overline{I}$ from $I_1$, $J_1$ to $x_1$, $y_1$, and a vector $\overline{J}$ from $I_2$, $J_2$ to $x_2$, $y_2$ may be represented as a Formula 1.

[Formula 1]

$$\overline{U} = \left\| \begin{bmatrix} x_1 \\ y_1 \end{bmatrix} - \begin{bmatrix} u_1 \\ v_1 \end{bmatrix} \right\|$$

$$\overline{V} = \left\| \begin{bmatrix} x_2 \\ y_2 \end{bmatrix} - \begin{bmatrix} u_2 \\ v_2 \end{bmatrix} \right\|$$

$$\overline{I} = \left\| \begin{bmatrix} x_1 \\ y_1 \end{bmatrix} - \begin{bmatrix} I_1 \\ J_1 \end{bmatrix} \right\|$$

$$\overline{J} = \left\| \begin{bmatrix} x_2 \\ y_2 \end{bmatrix} - \begin{bmatrix} I_2 \\ J_2 \end{bmatrix} \right\|$$

Meanwhile, a straight line $\tilde{U}$ including U1, V1 and $x_1$, $y_1$, a straight line $\tilde{V}$ including $U_2$, $V_2$ and $x_2$, $y_2$, a straight line $\tilde{I}$ including $I_1$, $J_1$ and $x_1$, $y_1$, and a straight line $\tilde{J}$ including $I_2$, $J_2$ and $x_2$, $y_2$ may be represented as a Formula 2.

[Formula 2]

$$\tilde{u} = \overline{U} \cdot t_1$$

$$\tilde{v} = \overline{V} \cdot t_2$$

$$\tilde{I} = \overline{I} \cdot p_1 + \begin{bmatrix} I_1 \\ J_1 \end{bmatrix}$$

$$\tilde{J} = \overline{J} \cdot p_2 + \begin{bmatrix} I_2 \\ J_2 \end{bmatrix}$$

Herein, $t_1$, $t_2$ are values which determine the length.

Meanwhile, $\tilde{u}$, $\tilde{v}$, $\overline{I}$, $\overline{J}$ may be represented as a Formula 3.

[Formula 3]

$$\tilde{u} = \overline{U} \cdot t_1 = \begin{bmatrix} u_x \\ v_y \end{bmatrix} = \tilde{I} = \overline{I} \cdot p_1 + \begin{bmatrix} I_1 \\ J_1 \end{bmatrix} = \begin{bmatrix} I_x \\ I_y \end{bmatrix} \Rightarrow \overline{I} \cdot p_1 = \overline{U} \cdot t_1 - \begin{bmatrix} I_1 \\ J_1 \end{bmatrix} \Rightarrow \overline{I} = \dfrac{\overline{U} \cdot t_1 - \begin{bmatrix} I_1 \\ J_1 \end{bmatrix}}{\left\| \overline{U} \cdot t_1 - \begin{bmatrix} I_1 \\ J_1 \end{bmatrix} \right\|}$$

$$\tilde{v} = \overline{V} \cdot t_1 = \begin{bmatrix} u_x \\ v_y \end{bmatrix} = \tilde{J} = \overline{J} \cdot p_2 + \begin{bmatrix} I_2 \\ J_2 \end{bmatrix} = \begin{bmatrix} J_x \\ J_y \end{bmatrix} \Rightarrow \overline{J} \cdot p_2 = \overline{V} \cdot t_2 - \begin{bmatrix} I_2 \\ J_2 \end{bmatrix} \Rightarrow \overline{J} = \dfrac{\overline{V} \cdot t_2 - \begin{bmatrix} I_2 \\ J_2 \end{bmatrix}}{\left\| \overline{V} \cdot t_2 - \begin{bmatrix} I_2 \\ J_2 \end{bmatrix} \right\|}$$

And referring to FIGS. 7 and 8, a coordinate $x_1$, $y_1$ which is the position that the light emitted from the first light source 150 (Referring to FIG. 7) or the second light source 151 (Referring to FIG. 7) is reflected by the first marker 110 may be within a radius r with a center portion x, y, a square of radius r may be represented as a Formula 4 since a summation of the vector which in inputted to $x_1$, $y_1$ is identical to a direction of a vector which connects $x_1$, $y_1$ with x, y which is a center portion of the first marker 110, a vector $\overline{P}$ from x, y to $x_1$, $y_1$, and a vector $\overline{Q}$ from x, y, to $x_2$, $y_2$ may be represented as a Formula 5.

[Formula 4]

$$(X - x)^2 + (Y - y)^2 = r^2$$

$$(\overline{U_x} \cdot t_1 - x)^2 + (\overline{U_y} \cdot t_1 - y)^2 = r^2$$

$$(\overline{V_x} \cdot t_2 - x)^2 + (\overline{V_y} \cdot t_2 - y)^2 = r^2$$

[Formula 5]

$$\overline{P} = \begin{bmatrix} x_1 \\ y_1 \end{bmatrix} - \begin{bmatrix} x \\ y \end{bmatrix} = \overline{U} \cdot t_1 - \begin{bmatrix} x \\ y \end{bmatrix} \qquad \overline{P} \times \dfrac{\overline{U} + \overline{I}}{2} = 0$$

$$\overline{Q} = \begin{bmatrix} x_2 \\ y_2 \end{bmatrix} - \begin{bmatrix} x \\ y \end{bmatrix} = \overline{V} \cdot t_2 - \begin{bmatrix} x \\ y \end{bmatrix} \qquad \overline{Q} \times \dfrac{\overline{V} + \overline{J}}{2} = 0$$

Meanwhile, and an error E of x, y, $t_1$, and $t_2$ may be represented as a Formula 6 by using the four equations included in the Formula 4 and Formula 5.

[Formula 6]

$$E = \left| (\overline{U_x} \cdot t_1 - x)^2 + (\overline{U_y} \cdot t_1 - y)^2 - r^2 \right| + \left| (\overline{V_x} \cdot t_2 - x)^2 + (\overline{V_y} \cdot t_2 - y)^2 - r^2 \right| + \left| \overline{P} \times \dfrac{\overline{U} + \overline{I}}{2} \right| + \left| \overline{Q} \times \dfrac{\overline{V} + \overline{J}}{2} \right|$$

Since x, y, $t_1$, and $t_2$ are parameters of the Formula 6, the two-dimensional central coordinate of the first marker 110 is calculated by a processor 140.

Two-dimensional central coordinates of second and third markers 111 and 112 are calculated by the processor 140 by repeating the process described above.

Meanwhile, a process of calculating a coordinate of a reflection position of a light which is emitted from the first light source 150 or the second light source 151 and reflected on a surface of a first marker 110 is described below.

As shown in FIG. 9, a radius of a first marker 110 is defined as r, a center portion of a lens portion 120 is defined as (0, 0), a position of a first light source 150 is defined as (0, d), a center portion of a first marker 110 (e, f), and a reflection position coordinate (x, y) in which a light emitted from the first light source 150 is reflected on a surface of a first marker 110.

[Formula 7]

$$(x-e)^2 + (y-f)^2 = r^2 \ \ -------------------------- (1)$$

$$y=(\tan\theta-\tan\theta')/(1+\tan\theta\tan\theta')x-(\tan\theta-\tan\theta')/(1+\tan\theta\tan\theta')+f \ \ ---- (2)$$

$$y=\tan\theta x \ \ ------------------------------ (3)$$

$$y=((\tan\theta-(2\tan\theta'/(1-\tan\theta'^2)))/(1+\tan\theta(2\tan\theta')/(1-\tan\theta'^2))))x+d \ \ --- (4)$$

Therefore, a square of a radius of a first marker 110 may be represented as equation (1) of Formula 7, and equation (1) of Formula 7 may be represented as equations (2) - (4) of Formula 7, and a coordinate of y-axis of a reflection position in which a light emitted from a first light source 150 is reflected on a surface of a first marker 110 may be obtained by solving the equation (4) of Formula 7.

Meanwhile, a coordinate of x-axis of a reflection position in which a light emitted from a first light source 150 is reflected on a surface of a first marker 110 may be obtained by solving the equation (1) of the Formula 7 for the variable x.

Therefore, coordinates of reflection positions in which lights emitted from first and second light sources 150 and 151 and reflected on surfaces of a first to third makers 110, 111, and 112 are calculated by repeating the process described above.

Then, three-dimensional coordinates of the first to third markers 110, 111, and 112 are calculated through the processor 140 by using the two dimensional coordinates of the first to third markers 110, 111, and 112 (S142).

As described above, after calculating the three-dimensional coordinates of the makers 110, 111, and 112, spatial position information and direction information of a target 200 are calculated by comparing the three-dimensional coordinates of each of the markers 110, 111, and 112 with geometric information, which is pre-stored in the processor 140, between the markers 110, 111, and 112 adjacent to each other (S150).

Herein, the geometric information between the markers 110, 111, and 112, adjacent to each other may be length information L1, L2, and L3 virtually connecting the markers 110, 111, and 112 adjacent to each other and angle information A1, A2, and A3 formed by a pair of the straight lines which connect the markers 110, 111, and 112 adjacent to each other.

In other words, spatial position information and direction information of a target 200 are calculated through the processor 140 by comparing three-dimensional coordinates of each of the markers 110, 111, and 112 with length information L1, L2, and L3 virtually connecting the markers 110, 111, and 112 adjacent to each other and angle information A1, A2, and A3 formed by a pair of the straight lines which connect the markers 110, 111, and 112 adjacent to each other, which are pre-stored in the processor 140.

As described above, in a tracking system and a method using the same according to an embodiment of the present invention, one light source of a pair of light sources 150 and 151 which is positioned different to each other directly emits a light toward a specific point of a surface of each of the markers 110, 111, and 112 and reflects the light toward a lens portion 120 such that first images of each of markers 110, 111, and 112 are formed on an image forming unit 130, the other light source emits a light such that the light is emitted toward a center of each of the markers 110, 111, and 112 through a lens portion 120 and the light is re-reflected toward a lens portion 120, and second images of each of makers 110, 111, and 112 are formed on an image forming unit 130, and therefore, a pair of images of each of markers 110, 111, and 112 are formed on an image forming unit 140.

In other words, three-dimensional coordinates of each of markers 110, 111, and 112 are calculated by using only one image forming unit 130 through a trigonometry since a pair of images of markers for each of markers 110, 111, and 112 are formed on an image forming unit 130 positioned different to each other.

Therefore, in a tracking system and a method using the same according to an embodiment of the present invention, spatial position information and direction information of markers 110, 111, and 113 which are attached on a target 200 are calculated by using only one image forming unit 130.

Therefore, there is an effect of reducing a manufacturing cost of the tracking system with small and lightweight, and relatively low restriction of surgical space comparing with conventional tracking system.

It will be apparent to those skilled in the art that various modifications and variation can be made in the present invention without departing from the scope of the invention. Thus, it is intended that the present invention cover the modifications and variations of this invention provided they come within the scope of the appended claims and their equivalents.

**Claims**

1. A tracking system comprising:

   at least three markers attached on a target to reflect lights;
   a pair of light sources positioned at different position to emit lights toward the markers;
   a lens portion passing the lights which are emitted from the pair of the light sources and reflected by the markers;
   an image forming unit to form a pair of marker images for each marker by receiving the lights passed by the lens portion; and
   a processor calculating three-dimensional coordinates of the markers by using the pair of marker images formed on the image forming unit for each marker, and calculating spatial position information and direction information of the target by comparing the three-dimensional coordinates of the markers and pre-stored geometric information between the markers adjacent to each other.

2. The tracking system of claim 1, further comprising a beam splitter arranged between the lens portion and the image forming unit to partially reflect the light emitted from one of the pair of the light sources toward a center of the markers through the lens portion and to partially pass the light, which is emitted toward the center of the markers, re-reflected by the markers, and flowed through the lens portion, to the image forming unit.

3. The tracking system of claim 2, further comprising a diffuser arranged between the light source, which emits the light toward the beam splitter, and the beam splitter to diffuse the light emitted from the light source.

4. The tracking system of claim 1, wherein the image forming unit is a camera which forms a pair of images for each marker by receiving lights which are reflected by the markers and pass the lens portion and the beam splitter, sequentially.

5. The tracking system of claim 1, wherein the geometric information between the markers comprises length information of straight lines which connect the markers adjacent to each other, and angle information which is formed by a pair of the straight lines adjacent to each other.

6. A tracking method comprising:

   emitting lights from a pair of light sources which are positioned at different position to each other toward at least three markers;
   reflecting the lights emitted from the pair of the light sources toward a lens portion by the markers;
   forming a pair of marker images on an image forming unit for each marker through the lights reflected from the markers passing the lens portion;
   calculating three-dimensional coordinates for each marker through a processor by using the pair of marker images formed on the image forming unit for each marker; and
   calculating spatial position and direction of the target by comparing the three-dimensional coordinates of each marker with geometric information, which is pre-stored in the processor, between the markers adjacent to each other.

7. The tracking method of claim 6, wherein one light source of the light sources emits the light toward the beam splitter arranged between the lens portion and the image forming unit, and emits the light toward a center of the markers through the lens portion by reflecting a portion of the light by the beam splitter, and the other light source directly emits the light toward the markers.

8. The tracking method of claim 7, wherein the light emitted toward the beam splitter is diffused by a diffuser arranged between the beam splitter, and emitted toward the beam splitter.

9. The tracking method of claim 6, wherein the geometric information between the markers comprises length information of straight lines which connect the markers adjacent to each other and angle information which is formed by a pair of the straight lines adjacent to each other.

10. The tracking method of claim 6, wherein calculating three-dimensional coordinates of the markers further comprises:

calculating two-dimensional central coordinates for each marker through the processor by using image formation position information of the pair of marker images formed on the image forming unit for each marker; and calculating three-dimensional coordinates of the markers by using the two-dimensional central coordinates of each marker.

EP 2 959 857 A1

## FIG. 1

## FIG. 2

# FIG. 3

Start

↓

emitting light from a pair of light
sources toward markers — S110

↓

reflecting lights, which are emitted from the pair
of light sources, toward lens portions by markers — S120

↓

forming a pair of marker images on image forming
unit for each marker through lights reflected
by markers and passing lens portion — S130

↓

calculating three-dimensional coordinates
for each marker through processor by using
the pair of marker images formed on image
forming unit for each marker — S140

↓

calculating spatial position and direction of
target through processor by comparing the
three-dimensional coordinates of each marker
with geometric information between markers — S150

↓

End

# FIG. 4

S140

```
        ┌─────────────┐
        │    Start    │
        └─────────────┘
               │
               ▼
┌──────────────────────────────────────────┐
│ calculating two-dimensional central       │
│ coordinates for each marker by using image│      S141
│ formation position information of the pair │
│ of marker images formed on image forming  │
│ unit for each marker                       │
└──────────────────────────────────────────┘
               │
               ▼
┌──────────────────────────────────────────┐
│ calculating three-dimensional coordinates │
│ for each marker through processor by using│      S142
│ the two-dimensional central coordinates   │
│ of each marker                             │
└──────────────────────────────────────────┘
               │
               ▼
        ┌─────────────┐
        │     End     │
        └─────────────┘
```

FIG. 5

EP 2 959 857 A1

# FIG. 6

FIG. 7

EP 2 959 857 A1

FIG. 8

$$\widetilde{I} = \overline{I} \cdot p_1 + \begin{bmatrix} I_1 \\ J_1 \end{bmatrix}$$

110

$x_1, y_1$

$\widetilde{u} = \overline{U} \cdot t_1$

$r$

$\overline{P}$

$x, y$

# FIG. 9

## INTERNATIONAL SEARCH REPORT

| International application No. |
| --- |
| **PCT/KR2014/000979** |

| A. CLASSIFICATION OF SUBJECT MATTER |
| --- |
| *A61B 19/00(2006.01)i, A61B 17/00(2006.01)i* |
| According to International Patent Classification (IPC) or to both national classification and IPC |

| B. FIELDS SEARCHED |
| --- |
| Minimum documentation searched (classification system followed by classification symbols) <br> A61B 19/00; A61B 5/05; G02B 5/12; G01B 11/00; A61B 1/04; B25J 19/00; A61B 17/00 |
| Documentation searched other than minimum documentation to the extent that such documents are included in the fields searched <br> Korean Utility models and applications for Utility models: IPC as above <br> Japanese Utility models and applications for Utility models: IPC as above |
| Electronic data base consulted during the international search (name of data base and, where practicable, search terms used) <br> eKOMPASS (KIPO internal) & Keywords: surgical robot, tracking system, marker, laparoscope |

| C. DOCUMENTS CONSIDERED TO BE RELEVANT |  |  |
| --- | --- | --- |
| Category* | Citation of document, with indication, where appropriate, of the relevant passages | Relevant to claim No. |
| A | JP 2007-130398 A (TOSHIBA CORP et al.) 31 May 2007 <br> See figures 1-6, claims 1, 14, paragraphs 0018-0027 | 1-10 |
| A | KR 10-2011-0118640 A (INTUITIVE SURGICAL OPERATIONS, INC.) 31 October 2011 <br> See claims 1, 13, paragraphs 0054-0067 | 1-10 |
| A | US 2007-0183041 A1 (MCCLOY, Bradley J. et al.) 09 August 2007 <br> See abstract, figures 2-3, paragraph 0020 | 1-10 |
| A | US 2005-0015005 A1 (ALFONS, Kockro Ralf ) 20 January 2005 <br> See abstract, figures 16-18, claims 1, 5 | 1-10 |

| ☐ Further documents are listed in the continuation of Box C. | ☒ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 25 JUNE 2014 (25.06.2014) | **26 JUNE 2014 (26.06.2014)** |

| Name and mailing address of the ISA/KR <br> Korean Intellectual Property Office <br> Government Complex-Daejeon, 189 Seonsa-ro, Daejeon 302-701, Republic of Korea <br> Facsimile No. 82-42-472-7140 | Authorized officer <br><br> Telephone No. |
| --- | --- |

Form PCT/ISA/210 (second sheet) (July 2009)

INTERNATIONAL SEARCH REPORT
Information on patent family members

International application No.

**PCT/KR2014/000979**

| Patent document cited in search report | Publication date | Patent family member | Publication date |
|---|---|---|---|
| JP 2007-130398 A | 31/05/2007 | JP 4459155 B2 | 28/04/2010 |
| KR 10-2011-0118640 A | 31/10/2011 | CN 102341054 A | 01/02/2012 |
| | | EP 2391289 A1 | 07/12/2011 |
| | | US 2010-0168763 A1 | 01/07/2010 |
| | | WO 2010-078016 A1 | 08/07/2010 |
| US 2007-0183041 A1 | 09/08/2007 | CN 101379412 A | 04/03/2009 |
| | | CN 101379412 B | 10/08/2011 |
| | | DE 112007000340 T5 | 18/12/2008 |
| | | US 7945311 B2 | 17/05/2011 |
| | | WO 2007-090288 A1 | 16/08/2007 |
| US 2005-0015005 A1 | 20/01/2005 | CA 2523727 A1 | 06/01/2005 |
| | | EP 1617779 A1 | 25/01/2006 |
| | | JP 2007-512854 A | 24/05/2007 |
| | | JP 2007-512854 T | 24/05/2007 |
| | | US 7491198 B2 | 17/02/2009 |
| | | WO 2005-000139 A1 | 06/01/2005 |

Form PCT/ISA/210 (patent family annex) (July 2009)